# EUROPEAN PATENT SPECIFICATION

(11) **EP 1 434 538 B1**
(45) Date of publication and mention of the grant of the patent: **01.08.2007**
(21) Application number: 02732077.9
(22) Date of filing: 17.06.2002
(51) Int. Cl.: A61F 2/06

(54) **A DELIVERY APPARATUS FOR A SELF-EXPANDING STENT**
EINFÜHRUNGSVORRICHTUNG FÜR SELBSTAUFDEHNBAREN STENT
APPAREIL DE POSE DESTINE A UNE ENDOPROTHESE A EXPANSION AUTOMATIQUE

(30) Priority: 19.06.2001 US 884728
(43) Date of publication of application: 07.07.2004
(73) Proprietor: Cordis Corporation, Miami Lakes, Florida 33014 (US)
(72) Inventor: DWYER, Clifford, J., Weston, FL 33331 (US); FELLER, Frederick, Margate, FL 33063 (US); JOHNSON, Kirk, Weston, FL 33326 (US); WILSON, David, J., Branchburg NJ 08876 (US)
(74) Representative: Mercer, Christopher Paul
(86) International application number: PCT/US2002/019203
(87) International publication number: WO 2002/102284

(56) References cited:
- EP-A- 0 941 716
- EP-A- 1 025 813
- US-A- 6 126 685

## Description

### FIELD OF THE INVENTION

The present invention relates to stents for use within a body passageway or duct which are particularly useful for repairing blood vessels narrowed or occluded by disease, and more particularly, to systems for delivering such stents.

### BACKGROUND OF THE INVENTION

Various endoprosthesis assemblies, which include expandable stents, have been proposed or developed for use in association with angioplasty treatments and other medical procedures. The endoprosthesis assembly is percutaneously routed to a treatment site and the stent is expanded to maintain or restore the patency of a body passageway such as a blood vessel or bile duct. A stent is typically cylindrical in shape comprising an expandable open frame. The stent will typically expand either by itself (self-expanding stents) or will expand upon exertion of an outwardly directed radial force on an inner surface of the stent frame by a balloon catheter or the like.

Stents for endovascular implantation into a blood vessel or the like, to maintain or restore the patency of the passageway, have been deployed percutaneously to minimize the invasiveness associated with surgical exposure of the treatment site during coronary artery bypass. Percutaneous deployment is initiated by an incision into the vascular system of the patient, typically into the femoral artery. A tubular or sheath portion of an introducer is inserted through the incision and extends into the artery. The introducer has a central lumen which provides a passageway through the patient's skin and artery wall into the interior of the artery. An outwardly tapered hub portion of the introducer remains outside the patient's body to prevent blood from leaking out of the artery along the outside of the sheath. The introducer lumen includes a valve to block blood flow out of the artery through the introducer passageway. A distal end of a guide wire is passed through the introducer passageway into the patient's vasculature. The guide wire is threaded through the vasculature until the inserted distal end extends just beyond the treatment site. The proximal end of the guide wire extends outside the introducer.

For endovascular deployment, a stent, in an unexpanded or constricted configuration, is crimped onto a deflated balloon portion of a balloon catheter. The balloon portion is normally disposed near a distal end of the balloon catheter. The catheter has a central lumen extending its entire length. The distal end of the balloon catheter is threaded onto the proximal end of the guide wire. The distal end of the catheter is inserted into the introducer lumen and the catheter is pushed along the guide wire until the stent reaches the treatment site. At the treatment site, the balloon is inflated causing the stent to radially expand and assume an expanded configuration. When the stent is used to reinforce a portion of the blood vessel wall, the stent is expanded such that its outer diameter is approximately ten percent to twenty percent larger than the inner diameter of the blood vessel at the treatment site, effectively causing an interference fit between the stent and the blood vessel that inhibits migration of the stent. The balloon is deflated and the balloon catheter is withdrawn from the patient's body. The guide wire is similarly removed. Finally, the introducer is removed from the artery.

An example of a commonly used stent is given in U.S. Patent 4,733,665 filed by Palmaz on November 7, 1985. Such stents are often referred to as balloon expandable stents. Typically the stent is made from a solid tube of stainless steel. Thereafter, a series of cuts are made in the wall of the stent. The stent has a first smaller diameter which permits the stent to be delivered through the human vasculature by being crimped onto a balloon catheter. The stent also has a second or expanded diameter. The expanded diameter is achieved through the application, by the balloon catheter positioned in the interior of the tubular shaped member, of a radially outwardly directed force.

However, such "balloon expandable" stents are often impractical for use in some vessels such as superficial arteries, like the carotid artery. The carotid artery is easily accessible from the exterior of the human body. A patient having a balloon expandable stent made from stainless steel or the like, placed in their carotid artery might be susceptible to sever injury through day to day activity. A sufficient force placed on the patients neck, such as by falling, could cause the stent to collapse, resulting in injury to the patient. In order to prevent this, self-expanding stents have been proposed for use in such vessels. Self-expanding stents act similarly to springs and will recover to their expanded or implanted configuration after being crushed.

One type of self-expanding stent is disclosed in U.S. Patent 4,665,771. The disclosed stent has a radially and axially flexible, elastic tubular body with a predetermined diameter that is variable under axial movement of ends of the body relative to each other and which is composed of a plurality of individually rigid but flexible and elastic thread elements defining a radially self-expanding helix. This type of stent is known in the art as a "braided stent" and is so designated herein. Placement of such stents in a body vessel can be achieved by a device which comprises an outer catheter for holding the stent at its distal end, and an inner piston which pushes the stent forward once it is in position.

Other types of self-expanding stents use alloys such as Nitinol (Ni-Ti alloy) which have shape memory and/or superelastic characteristics in medical devices which are designed to be inserted into a patient's body. The shape memory characteristics allow the devices to be deformed to facilitate their insertion into a body lumen or cavity and then be heated within the body so that the device returns to its original shape. Superelastic characteristics on the other hand generally allow the metal to be deformed and restrained in the deformed condition to facilitate the insertion of the medical device containing the metal into a patient's body, with such deformation causing the phase transformation. Once within the body lumen the restraint on the superelastic member can be removed, thereby reducing the stress therein so that the superelastic member can return to its original un-deformed shape by the transformation back to the original phase.

Alloys having shape memory/superelastic characteristics generally have at least two phases. These phases are a martensite phase, which has a relatively low tensile strength and which is stable at relatively low temperatures, and an austenite phase, which has a relatively high tensile strength and which is stable at temperatures higher than the martensite phase.

When stress is applied to a specimen of a metal such as Nitinol exhibiting superelastic characteristics at a temperature above which the austenite is stable (i.e. the temperature at which the transformation of martensite phase to the austenite phase is complete), the specimen deforms elastically until it reaches a particular stress level where the alloy then undergoes a stress-induced phase transformation from the austenite phase to the martensite phase. As the phase transformation proceeds, the alloy undergoes significant increases in strain but with little or no corresponding increases in stress. The strain increases while the stress remains essentially constant until the transformation of the austenite phase to the martensite phase is complete. Thereafter, further increase in stress is necessary to cause further deformation. The martensitic metal first deforms elastically upon the application of additional stress and then plastically with permanent residual deformation.

If the load on the specimen is removed before any permanent deformation has occurred, the martensitic specimen will elastically recover and transform back to the austenite phase. The reduction in stress first causes a decrease in strain. As stress reduction reaches the level at which the martensite phase transforms back into the austenite phase, the stress level in the specimen will remain essentially constant (but substantially less than the constant stress level at which the austenite transforms to the martensite) until the transformation back to the austenite phase is complete, i.e. there is significant recovery in strain with only negligible corresponding stress reduction. After the transformation back to austenite is complete, further stress reduction results in elastic strain reduction. This ability to incur significant strain at relatively constant stress upon the application of a load and to recover from the deformation upon the removal of the load is commonly referred to as superelasticity or pseudoelasticity. It is this property of the material which makes it useful in manufacturing tube cut self-expanding stents. The prior art makes reference to the use of metal alloys having superelastic characteristics in medical devices which are intended to be inserted or otherwise used within a patient's body. See for example, U.S. Patent No. 4,665,905 to Jervis and U.S. Patent No. 4,925,445 to Sakamoto et al.

Designing delivery systems for delivering self-expanding stents has proven difficult. One example of a prior art self-expanding stent delivery system is shown in U.S. Patent No. 4,580,568 to Gianturco. This patent discloses a delivery apparatus which uses a hollow sheath, like a catheter. The sheath is inserted into a body vessel and navigated therethrough so that its distal end is adjacent the target site. The stent is then compressed to a smaller diameter and loaded into the sheath at the sheath's proximal end. A cylindrical flat end pusher, having a diameter almost equal to the inside diameter of the sheath is inserted into the sheath behind the stent. The pusher is then used to push the stent from the proximal end of the sheath to the distal end of the sheath. Once the stent is at the distal end of the sheath, the sheath is pulled back, while the pusher remain stationary, thereby exposing the stent and expanding it within the vessel.

However, delivering the stent through the entire length of the catheter may cause many problems, including possible damage to a vessel or the stent during its travel. ln addition, it is often difficult to design a pusher having enough flexibility to navigate through the catheter, but also enough stiffness to push the stent out of the catheter. Therefore, it was determined that preloading the stent into the distal and of the catheter, and then delivering the catheter through the vessel to the target site may be a better approach. In order to ensure proper placement of the stent within catheter, it is often preferred that the stent be pre-loaded at the manufacturing site. Except this in itself has posed some problems. Because the catheter exerts a significant force on the self-expanding stent which keeps it from expanding, the stent may tend to become imbedded within the wall of the catheter. When this happens, the catheter has difficulty sliding over the stent during delivery. This situation can result in the stent becoming stuck inside the catheter, or could damage the stent during delivery.

Another example of a prior art self-expanding stent delivery system is given in U.S. Patent No. 4,732,152 to Wallsten et al. This patent discloses a probe or catheter having a self-expanding stent pre-loaded into its distal end. The stent is first placed within a flexible hose and compressed before it is loaded into the catheter. When the stent is at the delivery site the catheter and hose are withdrawn over the stent so that it can expand within the vessel. However, withdrawing the flexible hose over the stent during expansion could also cause damage to the stent.

An example of a more preferred self-expanding stent delivery system can be found in U.S. Patent No. 6, 019, 778 to Wilson et al. and issued on February 1, 2000, corresponding to EP-A-0 941 716. The preamble of claim 1 is based on the disclosure of said document. While using such a device, it is essential for the stent delivery device to be able to navigate through tortuous vessels, lesions and previously deployed devices (stents). The delivery system must follow a guide wire with out overpowering the wire in the tortuous vessels. The guidewire, when entering a new path, needs to be flexible enough to bend such that it is angled with respect to the delivery device proximal thereto. Because the guidewire extends through the distal end of the delivery device, if the distal end of the delivery device is stiff, it will not bend with the guidewire and may prolapse the wire causing the guidewire to move its position to align itself with the distal end of the delivery device. This could cause difficulty in navigating the delivery system, and may also cause any debris dislodged during the procedure to flow upstream and cause a stroke.

Therefore, there has been a need for a self-expanding stent delivery system which better navigates tortuous passageways, and more easily and accurately deploys the stent within the target area. The present invention provides such a delivery device.

### SUMMARY OF THE INVENTION

The present invention overcomes the disadvantages associated with self-expanding stent deployment as briefly described above.

The present invention is directed to a delivery apparatus for a self-expanding stent as described in claim 1. The delivery apparatus comprises a shaft having a proximal end, a distal end, a guidewire lumen extending between the proximal and distal ends, and a stent bed proximate the distal end upon which the self-expanding stent is positioned. The delivery apparatus further comprises a sheath defining an interior volume. The sheath has a proximal end, a distal end, and an enlarged section proximate the distal end. The sheath being coaxially positioned over the shaft such that the enlarged section is aligned with the stent bed. The sheath being formed from an inner polymeric layer, an outer polymeric layer, and a flat wire reinforcement layer.

The delivery apparatus for a self-expanding stent of the present invention utilizes a sheath constructed in a manner that allows flexibility in navigating through tortuous vessels, provides pushability for navigating through tight passageways, and substantially prevents the stent from becoming embedded in the device. The apparatus utilizes a sheath constructed from two polymeric layers and a reinforcement layer sandwiched therebetween. The reinforcement layer is formed from flat metallic wire to ensure adequate strength with reduced profile.

### BRIEF DESCRIPTION OF DRAWINGS

The foregoing and other features and advantages of the invention will be apparent from the following, more particular description of preferred embodiments of the invention, as illustrated in the accompanying drawings.

Figure 1 is a simplified elevational view of a stent delivery apparatus made in accordance with the present invention.

Figure 2 is a view similar to that of Figure 1 but showing an enlarged view of the distal end of the apparatus having a section cut away to show the stent loaded therein.

Figure 3 is a simplified elevational view of the distal end of the inner shaft made in accordance with the present invention.

Figure 4 is a cross-sectional view of Figure 3 taken along lines 4-4.

Figures 5 through 9 are partial cross-sectional views of the apparatus of the present invention sequentially showing the deployment of the self-expanding stent within the vasculature.

Figure 10 is a simplified elevational view of a shaft for a stent delivery apparatus made in accordance with the present invention.
Figure 11 is a partial cross-sectional view of the shaft and sheath of the stent delivery apparatus as described in the prior art, see EP-A-0 941 716.

Figure 12 is a partial cross-sectional view of the shaft and modified sheath of the stent delivery system in accordance with the present invention.

### DETAILED DESCRIPTION OF THE INVENTION

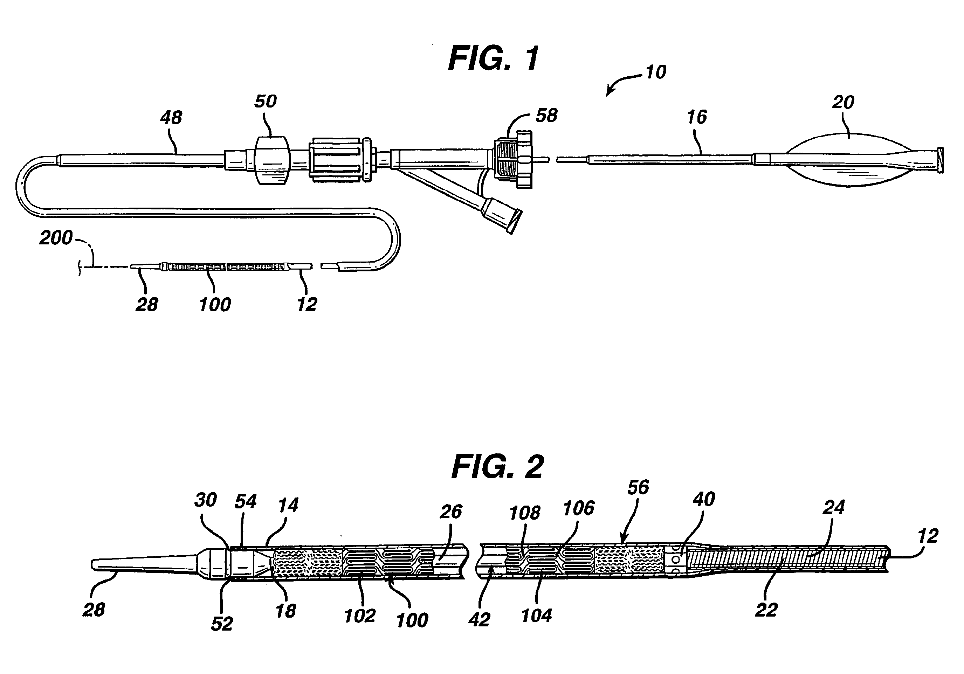

Figures 1 and 2 illustrate a self-expanding stent delivery apparatus 10 made in accordance with the present invention. Apparatus 10 comprises inner and outer coaxial tubes. The inner tube is called the shaft 12 and the outer tube is called the sheath 14. A self-expanding stent 100 is located within the sheath 14, wherein the stent 100 makes frictional contact with the sheath 14 and the shaft 12 is disposed coaxially within a lumen of the stent 100.

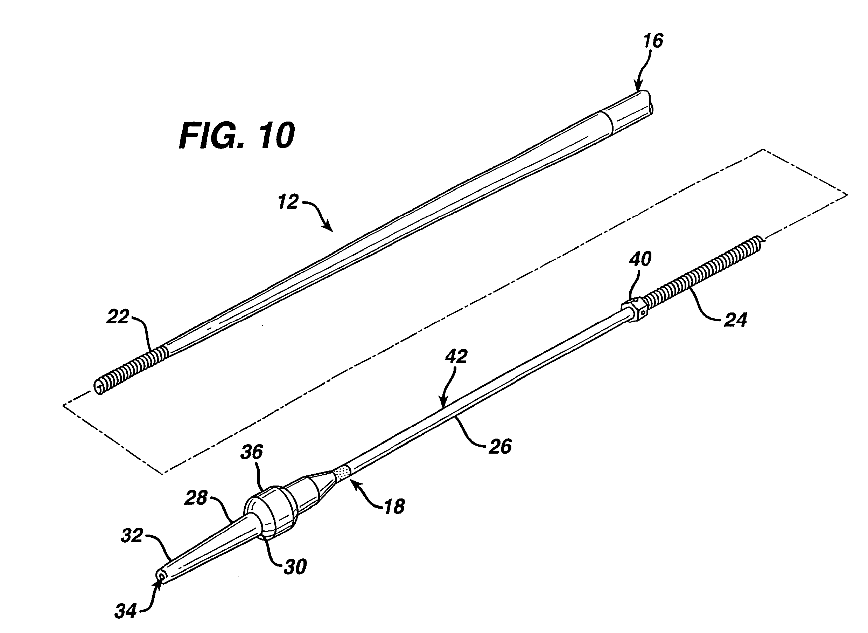

Shaft 12 has proximal and distal ends 16 and 18 respectively. The proximal end 16 of the shaft 12 has a Luer guidewire hub 20 attached thereto. As seen best from Figure 10, the proximal end 16 of the shaft 12 is preferably a ground stainless steel hypotube. In one exemplary embodiment, the hypotube is stainless steel and has a 1,07 mm (0.042 inch) outside diameter at its proximal end and then tapers to a 0,914 mm (0.036) inch outside diameter at its distal end. The inside diameter of the hypotube is 0,813 mm (0.032 inch) throughout its length. The tapered outside diameter is to gradually change the stiffness of the hypotube along its length. This change in the hypotube stiffness allows for a more rigid proximal end or handle end that is needed during stent deployment. If the proximal end is not stiff enough, the hypotube section extending beyond the Tuohy Borst valve described below could buckle as the deployment forces are transmitted. The distal end of the hypotube is more flexible allowing for better track-ability in tortuous vessels. The distal end of the hypotube also needs to be flexible to minimize the transition between the hypotube and the coil section described below.

As will be described in greater detail below, shaft 12 has a body portion 22, wherein at least a section thereof is made from a flexible coiled member 24, looking very much like a compressed or closed coil spring. Shaft 12 also includes a distal portion 26, distal to body portion 22, which is preferably made from a coextrusion of high-density polyethylene and nylon. The two portions 22 and 26 are joined together by any number of means known to those of ordinary skill in the art including heat fusing, adhesive bonding, chemical bonding or mechanical attachment.

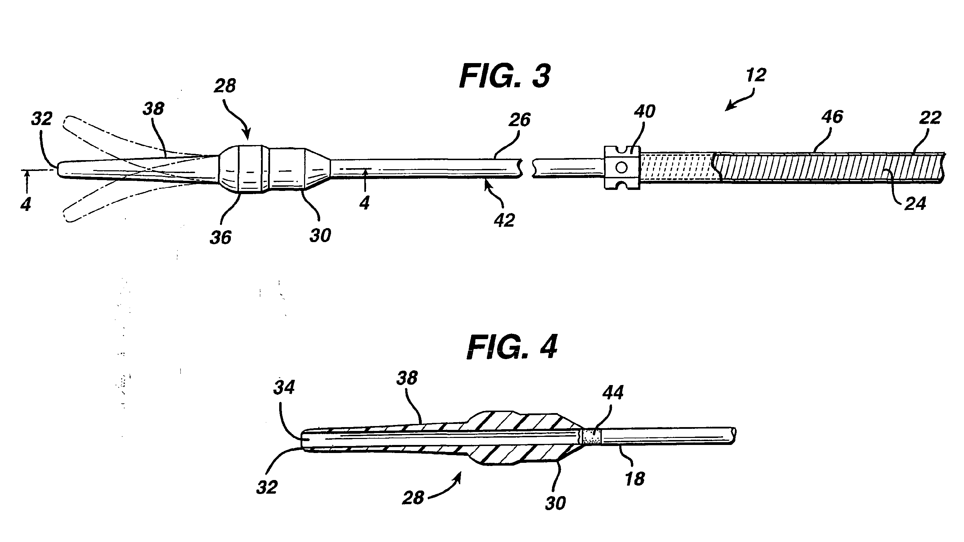

As best seen from Figure 3, the distal portion 26 of the shaft 12 has a distal tip 28 attached thereto. Distal tip 28 may be made from any number of suitable materials known in the art including polyamide, polyurethane, polytetrafluoroethylene, and polyethylene including multi-layer or single layer construction. The distal tip 28 has a proximal end 30 whose diameter is substantially the same as the outer diameter of the sheath 14 which is immediately adjacent thereto. The distal tip 28 tapers to a smaller diameter from its proximal end 30 to its distal end 32, wherein the distal end 32 of the distal tip 28 has a diameter smaller than the inner diameter of the sheath 14.

The stent delivery apparatus 10 glides over a guide wire 200 (shown in Figure 1) during navigation to the stent deployment site. As used herein, guidewire can also refer to similar guiding devices which have a distal protection apparatus incorporated herein. One preferred distal protection device is disclosed in WO-A-98/33443, having an international filing date of February 3, 1998. As discussed above, if the distal tip 28 is too stiff it will overpower the guide wire path and push the guide wire 200 against the lumen wall and in some very tortuous settings the stent delivery apparatus 10 could prolapse the wire. Overpowering of the wire and pushing of the apparatus against the lumen wall can prevent the device from reaching the target area because the guide wire will no longer be directing the device. Also as the apparatus is advanced and pushed against the lumen wall debris from the lesion can be dislodged and travel upstream causing complications to distal vessel lumens. The distal tip 28 is designed with an extremely flexible leading edge and a gradual transition to a less flexible portion. The distal tip 28 may be hollow and may be made of any number of suitable materials, including 40D nylon. Its flexibility may be changed by gradually increasing the thickness of its cross-sectional diameter, whereby the diameter is thinnest at its distal end, and is thickest at its proximal end. That is, the cross-sectional diameter and wall thickness of the distal tip 28 increases as you move in the proximal direction. This gives the distal end 32 of the distal tip 28 the ability to be directed by the guidewire prior to the larger diameter and thicker wall thickness (less flexible portion) of the distal tip 28 over-powering the guidewire. Over-powering the wire, as stated above, is when the apparatus (due to its stiffness) dictates the direction of the device instead of following the wire.

The guidewire lumen 34 has a diameter that is matched to hug the recommended size guide wire so that there is a slight frictional engagement between the guidewire 200 and the guidewire lumen 34 of distal tip 28. The distal tip 28 then has a rounded section 36 between its distal portion 32 and its proximal portion 30. This helps prevent the sheath 14 from slipping distally over the distal tip 28, and thereby exposing the squared edges of the sheath 14 to the vessel, which could cause damage thereto. This improves the device's "pushability." As the distal tip 28 encounters resistance it does not allow the sheath 14 to ride over it thereby exposing the sheath's 14 square cut edge. Instead the sheath 14 contacts the rounded section 36 of the distal tip 28 and thus transmits the forces applied to the distal tip 28. The distal tip 28 also has a proximally tapered section 38 which helps guide the distal tip 28 through the deployed stent 100 without providing a sharp edge that could grab or hang up on a stent strut end or other irregularity in the lumen inner diameter.

Attached to distal portion 26 of shaft 12 is a stop 40, which is proximal to the distal tip 28 and stent 100. Stop 40 may be made from any number of suitable materials known in the art, including stainless steel, and is even more preferably made from a highly radio-opaque material such as platinum, gold tantalum, or radio-opaque filled polymer. The stop 40 may be attached to shaft 12 by any suitable means, including mechanical or adhesive bonding, or by any other means known to those skilled in the art. Preferably, the diameter of stop 40 is large enough to make sufficient contact with the loaded stent 100 without making frictional contact with the sheath 14. As will be explained subsequently, stop 40 helps to "push" the stent 100 or maintain its relative position during deployment, by preventing the stent 100 from migrating proximally within the sheath 14 during retraction of the sheath 14 for stent deployment. The radio-opaque stop 40 also aides in positioning the stent 100 within the target lesion during deployment within a vessel, as is described below.

A stent bed 42 is defined as being that portion of the shaft 12 between the distal tip 28 and the stop 40 (Figure 2). The stent bed 42 and the stent 100 are coaxial so that the distal portion 26 of the shaft 12 comprising the stent bed 42 is located within the lumen of stent 100. The stent bed 42 makes minimal contact with stent 100 because of the space which exists between the shaft 12 and the sheath 14. As the stent 100 is subjected to temperatures at the austenite phase transformation it attempts to recover to its programmed shape by moving outwardly in a radial direction within the sheath 14. The sheath 14 constrains the stent 100 as will be explained in detail subsequently. Distal to the distal end of the loaded stent 100 attached to the shaft 12 is a radio-opaque marker 44 which may be made of platinum, iridium coated platinum, gold tantalum, stainless steel, radio-opaque filled polymer or any other suitable material known in the art.

As seen from Figures 2, 3 and 10, the body portion 22 of shaft 12 is made from a flexible coiled member 24, similar to a closed coil or compressed spring. During deployment of the stent 100, the transmission of compressive forces from the stop 40 to the Luer guidewire hub 20 is an important factor in deployment accuracy. A more compressive shaft 12 results in a less accurate deployment because the compression of the shaft 12 is not taken into account when visualizing the stent 100 under fluoroscopic imaging. However, a less compressive shaft 12 usually means less flexibility, which would reduce the ability of the apparatus 10 to navigate through tortuous vessels. A coiled assembly allows both flexibility and resistance to compression. When the apparatus 10 is navigating through the arteries, the shaft 12 is not in compression and therefore the coiled member 24 is free to bend with the delivery path. As one deploys the stent 100, tension is applied to the sheath 14 as the sheath 14 is retracted over the encapsulated stent 100. Because the stent 100 is self-expanding it is in contact with the sheath 14 and the forces are transferred along the stent 100 and to the stop 40 of the shaft 12. This results in the shaft 12 being under compressive forces. When this happens, the flexible coiled member 24 (no gaps between the coil members) transfers the compressive force from one coil to the next.

The flexible coiled member 24 further includes a covering 46 that fits over the flexible coiled member 24 to help resist buckling of the coiled member 24 in both bending and compressive modes. The covering 46 is an extruded polymer tube and is preferably a soft material that can elongate slightly to accommodate bending of the flexible coiled member 24, but does not allow the coils to ride over each other. Covering 46 may be made from any number of suitable materials including coextrusions of Nylon® and high-density polyethylene, polyurethane, polyamide, polytetrafluoroethylene, etc. The extrusion is also attached to the stop 40. Flexible coiled member 24 may be made of any number of materials known in the art including stainless steel, Nitinol, rigid polymers. In one exemplary embodiment, flexible coiled member 24 is made from a 0.076 mm (.003 inch) thick by 0,354 mm (.010 inch) wide stainless steel ribbon wire. The wire may be round, or more preferably flat to reduce the profile of the flexible coiled member 24.

Sheath 14 is preferably a polymeric catheter and has a proximal end 48 terminating at a sheath hub 50 (Figure 1). Sheath 14 also has a distal end 52 which terminates at the proximal end 30 of distal tip 28 of the shaft 12, when the stent 100 is in an un-deployed position as shown in Figure 2. The distal end 52 of sheath 14 includes a radio-opaque marker band 54 disposed along its outer surface (Figure 1). As will be explained below, the stent 100 is fully deployed when the marker band 54 is proximal to radio-opaque stop 40, thus indicating to the physician that it is now safe to remove the delivery apparatus 10 from the body.

As detailed in Figure 2, the distal end 52 of sheath 14 includes an enlarged section 56. Enlarged section 56 has larger inside and outside diameters than the inside and outside diameters of the sheath 14 proximal to enlarged section 56. Enlarged section 56 houses the pre-loaded stent 100, the stop 40 and the stent bed 42. The outer sheath 14 tapers proximally at the proximal end of enlarged section 56 to a smaller size diameter.

One particular advantage to the reduction in the size of the outer diameter of sheath 14 proximal to enlarged section 56 is in an increase in the clearance between the delivery apparatus 10 and the guiding catheter or sheath that the delivery apparatus 10 is placed through. Using fluoroscopy, the physician will view an image of the target site within the vessel, before and after deployment of the stent, by injecting a radio-opaque solution through the guiding catheter or sheath with the delivery apparatus 10 placed within the guiding catheter. Because the clearance between the sheath 14, and the guiding catheter is increased by tapering or reducing the outer diameter of the sheath 14 proximal to enlarged section 56, higher injection rates may be achieved, resulting in better images of the target site for the physician. The tapering of sheath 14 provides higher injection rates of radio-opaque fluid, both before and after deployment of the stent.

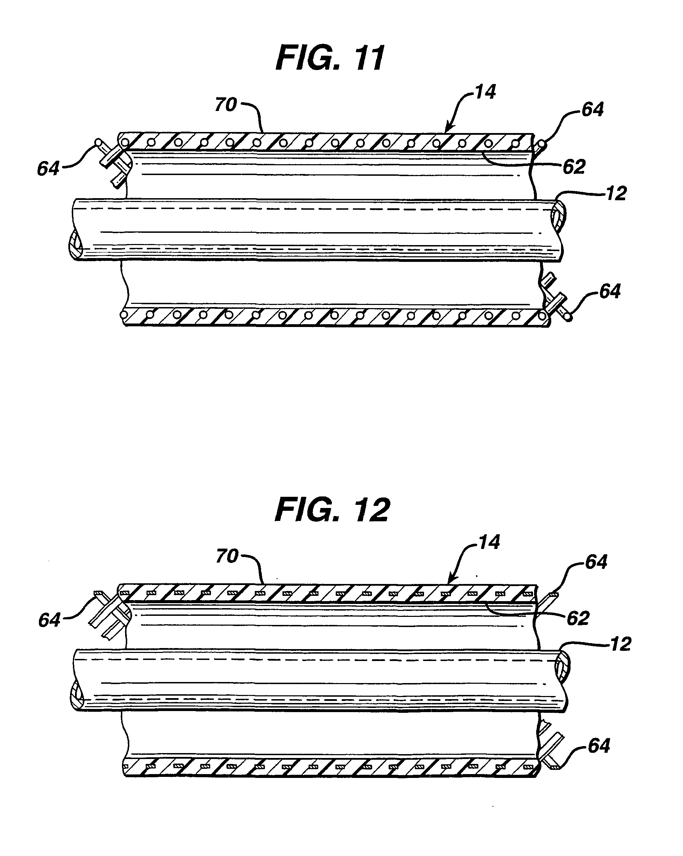

A problem encountered with earlier self-expanding stent delivery systems is that of the stent becoming embedded within the sheath in which it is disposed. Referring to Figure 11, there is illustrated a prior art sheath construction which may be effectively utilized to substantially prevent the stent from becoming embedded in the sheath as well as provide other benefits as described in detail below. As illustrated, the sheath 14 comprises a composite structure of at least two layers and preferably three layers. The outer layer 60 may be formed from any suitable biocompatible material. Preferably, the outer layer 60 is formed from a lubricious material for ease of insertion and removal of the sheath 14. In a preferred embodiment, the outer layer 60 comprises a polymeric material such as Nylon®. The inner layer 62 may also be formed from any suitable biocompatible material. For example, the inner layer 62 may be formed from any number of polymers including polyethylene, polyamide or polytetrafluroethylene. In a preferred embodiment, the inner layer 62 comprises polytetrafluroethylene. Polytetrafluroethylene is also a lubricious material which makes stent delivery easier, thereby preventing damage to the stent 100. The inner layer 62 may also be coated with another material to increase the lubricity thereof for facilitating stent deployment. Any number of suitable biocompatible materials may be utilized. ln an exemplary embodiment, silicone based coatings may be utilized. Essentially, a solution of the silicone based coating may be injected through the apparatus and allowed to cure at room temperature. The amount of silicone based coating utilized should be minimized to prevent transference of the coating to the stent 100. Sandwiched between the outer and inner layers 60 and 62, respectively, is a wire reinforcement layer 64. The wire reinforcement layer 64 may take on any number of configurations. In the exemplary embodiment, the wire reinforcement layer 64 comprises a simple under and over weave or braiding pattern. The wire used to form the wire reinforcement layer 64 may comprise any suitable material and any suitable cross-sectional shape. In the illustrated exemplary embodiment of the prior art, the wire forming the wire reinforcement layer 64 comprises stainless steel and has a substantially circular cross section. In order to function for its intended purpose, as described in detail below, the wire has a diameter of 0,051mm (0.002 inches).

The three layers 60, 62, and 64 comprising the sheath 14 collectively enhance stent deployment. The outer layer 60 facilitates insertion and removal of the entire apparatus 10. The inner layer 62 and the wire reinforcement layer 64 function to prevent the stent 100 from becoming embedded in the sheath 14. Self-expanding stents such as the stent 100 of the present invention tend to expand to their programmed diameter at a given temperature. As the stent attempts to undergo expansion, it exerts radially outward directed forces and may become embedded in the sheath 14 restraining it from expanding. Accordingly, the wire reinforcing layer 64 provides radial or hoop strength to the inner layer 62 thereby creating sufficient resistance to the outwardly directed radial force of the stent 100 within the sheath 14. The inner layer 62, also as discussed above, provides a lower coefficient of friction surface to reduce the forces required to deploy the stent 100 (typically in the range from about 2,3 to 3,6 kg, namely about five to eight pounds). The wire reinforcement layer 64 also provides tensile strength to the sheath 14. In other words, the wire reinforcement layer 64 provides the sheath 14 with better pushability, i.e., the ability to transmit a force applied by the physician at a proximal location on the sheath 14 to the distal tip 28, which aids in navigation across tight stenotic lesions within the vasculature. Wire reinforcement layer 64 also provides the sheath 14 with better resistance to elongation and necking as a result of tensile loading during sheath retraction for stent deployment.

The sheath 14 may comprise all three layers along its entire length or only in certain sections, for example, along the length of the stent 100. ln a preferred embodiment, the sheath 14 comprises all three layers along its entire length.

Because the size of typical self-expanding stents is large, as compared to balloon expandable coronary stents, the diameter or profile of the delivery devices therefor had to be large as well. However, it is always advantageous to have delivery systems which are as small as possible. This is desirable so that the devices can reach into smaller vessels and so that less trauma is caused to the patient. However, as stated above, the advantages of a thin reinforcing layer in a stent delivery apparatus outweighs the disadvantages of slightly increased profile.

In order to minimize the impact of the wire reinforcement layer on the profile of the apparatus 10, the configuration of the wire reinforcement layer 64 may be modified. For example, this may be accomplished in a number of ways, including changing the pitch of the braid, changing the shape of the wire, changing the wire diameter and/or changing the number of wires utilized. According to the present invention, the wire utilized to form the wire reinforcement layer is flat and may comprise a substantially rectangular cross-section as illustrated in Figure 12. In utilizing a substantially rectangular cross-section wire, the strength features of the reinforcement layer 64 may be maintained with a significant reduction in the profile of the delivery apparatus. The rectangular cross-section wire in the embodiment shown in Fig. 12 has a width of 0,076mm (0.003 inches) and a height of 0,025mm (0.001 inches). Accordingly, braiding the wire in a similar manner to Figure 11, results in a fifty percent decrease in the thickness of the wire reinforcement layer 64 while maintaining the same beneficial characteristics as the 0,051mm (0.002 inch) round wire of the prior art. The flat wire may comprise any suitable material, and preferably comprises stainless steel.

Figures 1 and 2 show the stent 100 as being in its fully un-deployed position. This is the position the stent is in when the apparatus 10 is inserted into the vasculature and its distal end is navigated to a target site. Stent 100 is disposed around the stent bed 42 and at the distal end 52 of sheath 14. The distal tip 28 of the shaft 12 is distal to the distal end 52 of the sheath 14. The stent 100 is in a compressed state and makes frictional contact with the inner surface of the sheath 14.

When being inserted into a patient, sheath 14 and shaft 12 are locked together at their proximal ends by a Tuohy Borst valve 58. This prevents any sliding movement between the shaft 12 and sheath 14, which could result in a premature deployment or partial deployment of the stent 100. When the stent 100 reaches its target site and is ready for deployment, the Tuohy Borst valve 58 is opened so that the sheath 14 and shaft 12 are no longer locked together.

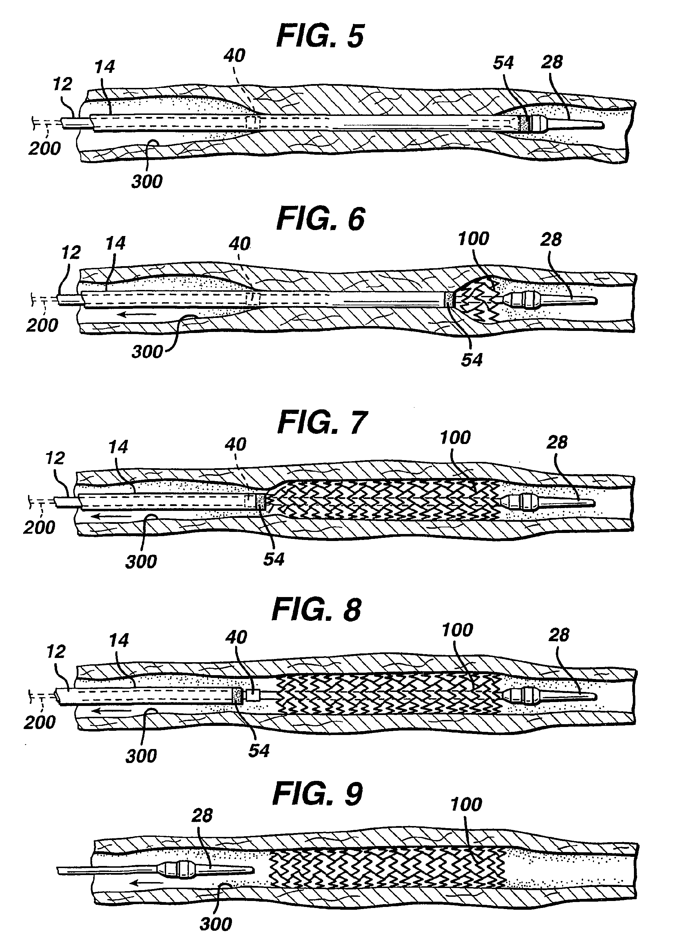

The method under which delivery apparatus 10 deploys stent 100 may best be described by referring to Figures 5-9. This method is just an example of use of the apparatus. In Figure 5, the delivery apparatus 10 has been inserted into a vessel 300 so that the stent bed 42 is at a target diseased site. Once the physician determines that the radio-opaque marker band 54 and stop 40 on shaft 12 indicating the ends of stent 100 are sufficiently placed about the target disease site, the physician would open Tuohy Borst valve 58. The physician would then grasp the Luer guidewire hub 20 of shaft 12 so as to hold shaft 12 in a fixed position. Thereafter, the physician would grasp the Tuohy Borst valve 58, attached proximally to sheath 14, and slide it proximal, relative to the shaft 12 as shown in Figures 6 and 7. Stop 40 prevents the stent 100 from sliding back with sheath 14, so that as the sheath 14 is moved back, the stent 100 is effectively "pushed" out of the distal end 52 of the sheath 14, or held in position relative to the target site. Stent 100 should be deployed in a distal to proximal direction to minimize the potential for creating emboli with the diseased vessel 300. Stent deployment is complete when the radio-opaque band 54 on the sheath 14 is proximal to radio-opaque stop 40, as shown in Figure 8. The apparatus 10 can now be withdrawn through stent 100 and removed from the patient.

Figures 2 and 9 show an example of a preferred embodiment of a stent 100, which may be used in conjunction with the present invention. Stent 100 which does not form part of the present invention is shown in its unexpanded compressed state, before it is deployed, in Figure 2. Stent 100 is preferably made from a superelastic alloy such as Nitinol. Most preferably, the stent 100 is made from an alloy comprising from about 50.5 percent (as used herein these percentages refer to atomic percentages) Ni to about 60 percent Ni, and most preferably about 55 percent Ni, with the remainder of the alloy Ti. Preferably, the stent 100 is such that it is superelastic at body temperature, and preferably has an Af in the range from about twenty-one degrees C to about thirty-seven degrees C. The superelastic design of the stent makes it crush recoverable which, as discussed above, can be used as a stent or frame for any number of vascular devices for different applications.

Stent 100 is a tubular member having front and back open ends a longitudinal axis extending there between. The tubular member has a first smaller diameter, Figure 2, for insertion into a patient and navigation through the vessels, and a second larger diameter for deployment into the target area of a vessel. The tubular member is made from a plurality of adjacent hoops 102 extending between the front and back ends. The hoops 102 include a plurality of longitudinal struts 104 and a plurality of loops 106 connecting adjacent struts, wherein adjacent struts are connected at opposite ends so as to form a substantially S or Z shape pattern. Stent 100 further includes a plurality of curved bridges 108, which connect adjacent hoops 102. Bridges 108 connect adjacent struts together at bridge to loop connection points which are offset from the center of a loop.

The above described geometry helps to better distribute strain throughout the stent, prevents metal to metal contact when the stent is bent, and minimizes the opening size between the features, struts, loops and bridges. The number of and nature of the design of the struts, loops and bridges are important factors when determining the working properties and fatigue life properties of the stent. Preferably, each hoop has between twenty-four to thirty-six or more struts. Preferably the stent has a ratio of number of struts per hoop to strut length (in inches) which is greater than two hundred. The length of a strut is measured in its compressed state parallel to the longitudinal axis of the stent.

In trying to minimize the maximum strain experienced by features, the stent utilizes structural geometries which distribute strain to areas of the stent which are less susceptible to failure than others. For example, one vulnerable area of the stent is the inside radius of the connecting loops. The connecting loops undergo the most deformation of all the stent features. The inside radius of the loop would normally be the area with the highest level of strain on the stent. This area is also critical in that it is usually the smallest radius on the stent. Stress concentrations are generally controlled or minimized by maintaining the largest radii possible. The stent tries to minimize local strain concentrations on the bridge and bridge to loop connection points. One way to accomplish this is to utilize the largest possible radii while maintaining feature widths which are consistent with applied forces. Another consideration is to minimize the maximum open area of the stent. Efficient utilization of the original tube from which the stent is cut increases stent strength and it's ability to trap embolic material.

Although shown and described is what is believed to be the most practical and preferred embodiments, it is apparent that departures from specific designs described and shown will suggest themselves to those skilled in the art and may be used without departing from the scope of the invention. The present invention is not restricted to the particular constructions described and illustrated, but should be constructed to cohere with all modifications that may fall within the scope of the appended claims.

## Claims

1. A delivery apparatus (10) for a self-expanding stent (100) comprising:
a shaft (12) having a proximal end (16), a distal end (18), a guidewire lumen (34) extending between the proximal (16) and distal (18) ends, and a stent bed (42) proximate the distal end (18) upon which the self-expanding stent (100) is positioned; and
a sheath (14) defining an interior volume, the sheath (14) having a proximal end (48), a distal end (52), and an enlarged section (56) proximate the distal end (52), the sheath (14) being coaxially positioned over the shaft (12) such that the enlarged section (56) is aligned with the stent bed (42) the sheath (14) being formed from an inner polymeric layer (62), an outer polymeric layer (70), and a wire reinforcement layer (64), **characterized in that** the wire of the wire reinforcement layer (54) is flat.

2. The delivery apparatus (10) for a self-expanding stent (100) according to Claim 1, wherein the wire reinforcement layer (64) is sandwiched between the inner (62) and outer (70) polymeric layers and extends along a predetermined length of the sheath (14).

3. The delivery apparatus (10) for a self-expanding stent (100) according to Claim 1 or claim 2 wherein the wire reinforcement layer (64) comprises wire having a substantially rectangular cross section.

4. The delivery apparatus (10) for a self-expanding stent (100) according to Claim 1, 2 or 3, wherein the wire comprises stainless steel and has cross-sectional dimensions of 0,076 mm (0.003 inches) by 0.025mm (0.001 inches).

5. The delivery apparatus (10) for a self-expanding stent (100) according to any preceding Claim, wherein the flat wire is arranged in a braided configuration.

6. The delivery apparatus (10) for a self-expanding stent (100) according to any preceding Claim, wherein the inner polymeric layer (62) comprises polytetrafluoroethylene.

7. The delivery apparatus (10) for a self-expanding stent (100) according to any preceding Claim, wherein the outer polymeric layer (70) comprises Nylon®.

8. The delivery apparatus (10) for a self-expanding stent (100) of any preceding claim, further comprising a lubricious coating on the inner polymeric layer (62).

9. The delivery apparatus (10) for a self-expanding stent (100) according to Claim 8, wherein the lubricious coating on the inner polymeric layer (62) comprises a silicone based material.

10. The delivery apparatus (10) for a self-expanding stent (100) according to any preceding claim, wherein the shaf (12) and the sheath (14) are each substantially tubular.

## Patentansprüche

1. Einführungsvorrichtung (10) für einen selbstexpandierenden Stent (100), die folgendes aufweist:
einen Schaft (12) mit einem proximalen Ende (16), einem distalen Ende (18), einem Führungsdrahtlumen (34), das sich zwischen dem proximalen Ende (16) und dem distalen Ende (18) erstreckt, und einem Stentbett (42) proximal zu dem distalen Ende (18), auf dem der selbstexpandierende Stent (100) angeordnet wird; und
eine Ummantelung (14), die ein inneres Volumen definiert, wobei die Ummantelung (14) ein proximales Ende (48), ein distales Ende (52) und einen vergrößerten Abschnitt (56) proximal zu dem distalen Ende (52) aufweist, und wobei die Ummantelung (14) koaxial über dem Schaft (12) so angeordnet ist, daß der vergrößerte Abschnitt (56) zu dem Stentbett (42) ausgerichtet ist, und die Ummantelung (14) aus einer inneren Polymerschicht (62), einer äußeren Polymerschicht (70) und einer Drahtverstärkungsschicht (64) gebildet ist, **dadurch gekennzeichnet, daß** der Draht der Drahtverstärkungsschicht (64) flach ist.

2. Einführungsvorrichtung (10) für einen selbstexpandierenden Stent (100) nach Anspruch 1, bei welcher die Drahtverstärkungsschicht (64) zwischen der inneren Polymerschicht (62) und der äußeren Polymerschicht (70) eingeschlossen ist und sich entlang einer vorbestimmten Länge der Ummantelung (14) erstreckt.

3. Einführungsvorrichtung (10) für einen selbstexpandierenden Stent (100) nach Anspruch 1 oder nach Anspruch 2, bei welcher die Drahtverstärkungsschicht (64) einen Draht mit einem im wesentlichen rechteckigen Querschnitt aufweist.

4. Einführungsvorrichtung (10) für einen selbstexpandierenden Stent (100) nach Anspruch 1, 2 oder 3, bei welcher der Draht rostfreien Stahl aufweist und Querschnittsabmessungen von 0,076 mm (0,003 Zoll) auf 0,025 mm (0,001 Zoll) aufweist.

5. Einführungsvorrichtung (10) für einen selbstexpandierenden Stent (100) nach einem der vorhergehenden Ansprüche, bei welcher der flache Draht in einer geflochtenen Konfiguration angeordnet ist.

6. Einführungsvorrichtung (10) für einen selbstexpandierenden Stent (100) nach einem der vorhergehenden Ansprüche, bei welcher die innere Polymerschicht (62) Polytetrafluorethylen aufweist.

7. Einführungsvorrichtung (10) für einen selbstexpandierenden Stent (100) nach einem der vorhergehenden Ansprüche, bei welcher die äußere Polymerschicht (70) Nylon® aufweist.

8. Einführungsvorrichtung (10) für einen selbstexpandierenden Stent (100) nach einem der vorhergehenden Ansprüche, die weiterhin eine gleitfähige Beschichtung auf der inneren Polymerschicht (62) aufweist.

9. Einführungsvorrichtung (10) für einen selbstexpandierenden Stent (100) nach Anspruch 8, bei welcher die gleitfähige Beschichtung auf der inneren Polymerschicht (62) ein auf Silikon beruhendes Material aufweist.

10. Einruhrungsvorrichtung (10) für einen selbstexpandierenden Stent (100) nach einem der vorhergehenden Ansprüche, bei welcher der Schaft (12) und die Ummantelung (14) jeweils im wesentlichen röhrenförmig sind.

## Revendications

1. Appareil de pose (10) destiné à une endoprothèse à expansion automatique (100) comprenant :
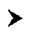 une tige (12) présentant une extrémité proximale (16), une extrémité distale (18), une lumière de fil de guidage (34) s'étendant entre les extrémités proximale (16) et distale (18), et une base d'endoprothèse (42) à proximité de l'extrémité distale (18) sur laquelle est positionnée l'endoprothèse à expansion automatique (100) ; et
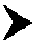 une gaine (14) définissant un volume intérieur, la gaine (14) présentant une extrémité proximale (48), une extrémité distale (52), et une section agrandie (56) à proximité de l'extrémité distale (52), la gaine (14) étant positionnée de manière coaxiale sur la tige (12) de sorte que la section agrandie (56) soit alignée sur la base d'endoprothèse (42), la gaine (14) étant formée d'une couche polymère intérieure (62), d'une couche polymère extérieure (70) et d'une couche de renforcement à fil (64), **caractérisé en ce que** le fil de la couche de renforcement à fil (64) est plat.

2. Appareil de pose (10) destiné à une endoprothèse à expansion automatique (100) selon la revendication 1, dans lequel la couche de renforcement à fil (64) est coincée entre les couches polymères intérieure (62) et extérieure (70) et s'étend sur une longueur prédéterminée de la gaine (14).

3. Appareil de pose (10) destiné à une endoprothèse à expansion automatique (100) selon la revendication 1 ou la revendication 2, dans lequel la couche de renforcement à fil (64) comprend un fil ayant une coupe transversale sensiblement rectangulaire.

4. Appareil de pose (10) destiné à une endoprothèse à expansion automatique (100) selon la revendication 1, 2 ou 3, dans lequel le fil comprend de l'acier inoxydable et a des dimensions en coupe transversale de 0,076 mm (0,003 pouce) sur 0,025 mm (0,001 pouce).

5. Appareil de pose (10) destiné à une endoprothèse à expansion automatique (100) selon l'une quelconque des revendications précédentes, dans lequel le fil plat est agencé selon une configuration tressée.

6. Appareil de pose (10) destiné à une endoprothèse à expansion automatique (100) selon l'une quelconque des revendications précédentes, dans lequel la couche polymère intérieure (62) comprend du polytétrafluoroéthylène.

7. Appareil de pose (10) destiné à une endoprothèse à expansion automatique (100) selon l'une quelconque des revendications précédentes, dans lequel la couche polymère extérieure (70) comprend du Nylon^{®}.

8. Appareil de pose (10) destiné à une endoprothèse à expansion automatique (100) selon l'une quelconque des revendications précédentes, comprenant en outre un revêtement lubrifiant sur la couche polymère intérieure (62).

9. Appareil de pose (10) destiné à une endoprothèse à expansion automatique (100) selon la revendication 8, dans lequel le revêtement lubrifiant sur la couche polymère intérieure (62) comprend un matériau à base de silicone.

10. Appareil de pose (10) destiné à une endoprothèse à expansion automatique (100) selon l'une quelconque des revendications précédentes, dans lequel la tige (12) et la gaine (14) sont chacune sensiblement tubulaires.
